**Europäisches Patentamt**

**European Patent Office**

**Office européen des brevets**

(19)

(11) Veröffentlichungsnummer: **0 022 128**
A1

(12) # EUROPÄISCHE PATENTANMELDUNG

(21) Anmeldenummer: 80890068.2

(22) Anmeldetag: 18.06.80

(51) Int. Cl.³: **G 01 N 33/92**
G 01 N 33/68, G 01 N 33/96

(30) Priorität: 27.06.79 AT 4484 79

(43) Veröffentlichungstag der Anmeldung:
07.01.81 Patentblatt 81'1

(84) Benannte Vertragsstaaten:
BE CH DE FR GB IT LI LU NL SE

(71) Anmelder: IMMUNO Aktiengesellschaft für
chemisch-medizinische Produkte
Industriestrasse 72
1220 Wien(AT)

(72) Erfinder: Seidel, Dietrich, Prof. Dr.
Schlosswolfsbrunnenweg 15
D-6900 Heidelberg(DE)

(72) Erfinder: Wieland, Heinrich, Dr.
Wiesenweg 2
D-3401 Waake(DE)

(72) Erfinder: Molinari, Ewald, Dr.
Brühlerstrasse 79
D-2340 Mödling(DE)

(74) Vertreter: Wolfram, Gustav, Dipl.Ing.
Schwindgasse 7 P.O.Box 205
A-1041 Wien(AT)

(54) Verfahren zum Konservieren der elektrophoretischen Eigenschaften von Lipoproteinen und Verwendung der konservierten Seren.

(57) Bei der Bestimmung des Lipoproteinmusters von Lipoproteinen, insbesondere von LDL-, VLDL- und HDL-Lipoproteinen in menschlichen oder tierischen Körperflüssigkeiten, wie Plasma, Plasmafraktionen oder Seren, ist die Schwierigkeit gegeben, daß schon bei kurzzeitiger Lagerung die native Zusammensetzung der Lipoproteine irreversibel verändert wird, sodaß die Bestimmung ungenaue bzw. verfälschte Resultate ergibt. Auch Kontrollseren unterliegen einer solchen Veränderung, sodaß es bisher nicht möglich war, die qualitativen und quantitativen elektrophoretischen Eigenschaften von Lipoproteinen in einem Kontrollserum zu bewahren.

Diese Schwierigkeiten werden behoben, indem den zu analysierenden Körperflüssigkeiten nicht reduzierende Zucker, insbesondere Saccharose, zugegeben werden, das Gemisch lyophilisiert und das Lyophilisat vor der Bestimmung rekonstituiert wird. Auf diese Weise werden die nativen elektrophoretischen Eigenschaften der Lipoproteine konserviert. Die auf solche Weise konservierten Körperflüssigkeiten können auch vorteilhaft als Kontrollseren für Plasma- bzw. Serumuntersuchungen verwendet werden.

./...

FIG.3

60,4%  |  13,7%  |  25,9%

Verfahren zum Konservieren der elektrophoretischen Eigenschaften von Lipoproteinen

Die Erfindung betrifft ein Verfahren zum Konservieren der elektrophoretischen Eigenschaften von Lipoproteinen, insbesondere von LDL-, VLDL- und HDL-Lipoproteinen in menschlichem oder tierischem Plasma, Plasmafraktionen oder Seren, in welchen das Lipoproteinmuster elektrophoretisch zu bestimmen ist.

Ein Verfahren zur Bestimmung von Lipoproteinmustern in menschlichen oder tierischen Körperflüssigkeiten ist in der AT-PS 320.865 beschrieben. Das Verfahren besteht darin, daß die in den Körperflüssigkeiten enthaltenen Lipoproteine in einem Trägermedium, wie einem Gel oder auf Folien elektrophoretisch aufgetrennt werden, wobei nach Eintritt des gewünschten Auftrennungseffektes der Träger mit einer Entwicklerlösung behandelt wird, die eine oder mehrere der folgenden Substanzen enthält: Polyanionen, wie Heparin oder Dextransulfat, Natriumdodezylsulfat, Natriumphosphowolframat, Natriumoleat, Natriumsalze von Gallensäuren, zweckmäßig in Anwesenheit von zweiwertigen Kationen, wie Magnesium und Kalzium sowie gegebenenfalls NaCl, wobei schwerlösliche Komplexsalze mit den Lipoproteinen gebildet werden.

Die Komplexsalze der einzelnen Lipoproteine bilden auf dem Trägermedium Banden, die quantitativ densitometrisch ausgewertet werden, wobei man ein genaues Bild über die quantitative Verteilung der verschiedenen Lipoproteine im Serum erhält.

Bestimmungen dieser Art sind insbesondere bei der Diagnose von verschiedenen Formen der Hyperlipoproteinämie notwendig. Die beschriebene Technik zur Bestimmung der Lipoproteinmuster erfordert jedoch einen ziemlichen Zeitaufwand, sodaß es vielfach, insbesondere bei Reihenuntersuchungen, nicht möglich ist, die Bestimmung unmittelbar oder kurz nach der Blutabnahme durchzuführen. Bei Lagerung von nur wenigen Tagen wird jedoch die native Zusammensetzung der Lipoproteine irreversibel verändert, was insbesondere für die unstabilen Lipoproteine LDL, VLDL und HDL gilt.

Bisherige Bemühungen, die Stabilität bzw. Lagerungsfähigkeit der zu untersuchenden Proben über einen längeren Zeitraum sicherzustellen, hatten bisher keinen zufriedenstellenden Erfolg. So hat sich z.B. gezeigt, daß eine Lyophilisierung nicht ausreicht. Auch in lyophilisierten Proben werden die instabilen Lipoproteine schon während kurzer Lagerung irreversibel verändert.

Das Bestreben, die native Zusammensetzung der Lipoproteine zu erhalten bzw. zu konservieren, ist insbesondere auch für die Qualitätssicherung von Kontrollseren gegeben, worüber in den meisten Ländern der Welt gesetzliche Vorschriften bestehen. Bisher war es nicht möglich, die qualitativen und quantitativen elektrophoretischen Eigenschaften von Lipoproteinen in menschlichen oder tierischen Körperflüssigkeiten in einem Kontrollserum zu bewahren, weshalb es bisher keine Möglichkeit gab, die Analyse von Lipoproteinspektren mit Kontrollseren abzusichern. Es besteht somit Bedarf nach einem Kontrollserum, das auf seinen natürlichen Lipoproteingehalt hin analysiert und über einen längeren Zeitraum in stabiler Form gehalten werden kann.

Die Erfindung bezweckt die Vermeidung der geschilderten Schwierigkeiten und stellt sich die Aufgabe, in Plasma, Plasmafraktionen oder Seren die intakte Molekülstruktur der Lipoproteine zu bewahren, bis sie elektrophoretisch aufgetrennt in ihrer nativen Form bestimmbar sind. Ein weiteres Ziel der Erfindung ist es, Kontrollseren, die Lipoproteine enthalten, vorzusehen, ihre Qualität zu sichern und damit die Möglichkeit der Kontrolle von elektrophoretischen Lipoprotein-Analysen zu schaffen.

Diese Aufgaben werden erfindungsgemäß dadurch gelöst, daß dem Plasma bzw. den Plasmafraktionen oder Seren nicht-reduzierende Zucker, insbesondere Saccharose, zugegeben werden, das Gemisch lyophilisiert und das Lyophilisat vor der Bestimmung rekonstituiert wird.

Vorteilhaft werden die nicht-reduzierenden Zucker in einer Menge von 10 bis 20 % w/v zugegeben.

Eine weitere bevorzugte Ausführungsform der Erfindung besteht darin, daß das Zucker enthaltende Gemisch vor der Lyophilisierung bei mindestens $-70^{o}C$ schockgefroren wird, worauf nach Erwärmen auf $-40^{o}C$ bis $-35^{o}C$ das Gemisch lyophilisiert wird.

Es ist zwar bereits in der US-PS 4,127,502 angegeben worden, daß eine Serum-Matrix oder eine von Serum abgeleitete Komposition, welche Lipide enthält, durch Zugabe von Zucker, Zuckeraminen oder Zuckeralkoholen und anschließende Lyophilisierung stabilisiert werden kann, wobei die rekonstituierte Mischung eine Abnahme der optischen Dichte erkennen läßt, doch befaßt sich dieser frühere Vorschlag nicht mit der Bewahrung der elektrophoretischen Eigenschaften von Lipoproteinen. Die in dem früheren Vorschlag erwähnten Lipide haben eine andere Zusammensetzung als die Lipoproteine. Sie enthalten keine Eiweißkomponenten, insbesondere keine Apoproteinanteile. Gerade die letzteren sind aber verantwortlich für den raschen Zerfall des nativen Lipoproteinmoleküls.

Zusätzlich zu der Möglichkeit, Lipoprotein enthaltende Untersuchungsproben längere Zeit zu lagern und ein verläßliches Kontrollserum für die Lipoproteindiagnostik zur Verfügung zu stellen, erlaubt das erfindungsgemäße Verfahren auch eine unverfälschte Analytik aller übrigen im Plasma vorhandenen Bestandteile, nämlich der Elektrolyte Natrium, Kalium, Calcium, des Kreatins, des Harnstoffes, des anorganischen Phosphats, des Eisens, der Lipide Cholesterin Triglyceride und Phospholipide der Harnsäure; weiters können die Enzymaktivitäten im Plasma, wie GOT, GPT, CPK, alkalische Phosphatase, saure Phosphatase, Gamma GT, Amylase, gemessen werden. Darüber hinaus ist auch eine Bestimmung der Glucose, des Bilirubins, des Gesamteiweißes sowie bestimmter Eiweißfraktionen, wie des Albumins, der Globuline und weiterer Subfraktionen ohne weiteres möglich.

Es hat sich gezeigt, daß erfindungsgemäß stabilisierte Seren, insbesondere Kontrollseren, mindestens ein Jahr auch bei Lagerung bei 37°C haltbar und unverändert bleiben.

Die Erfindung wird durch die Zeichnung näher erläutert:

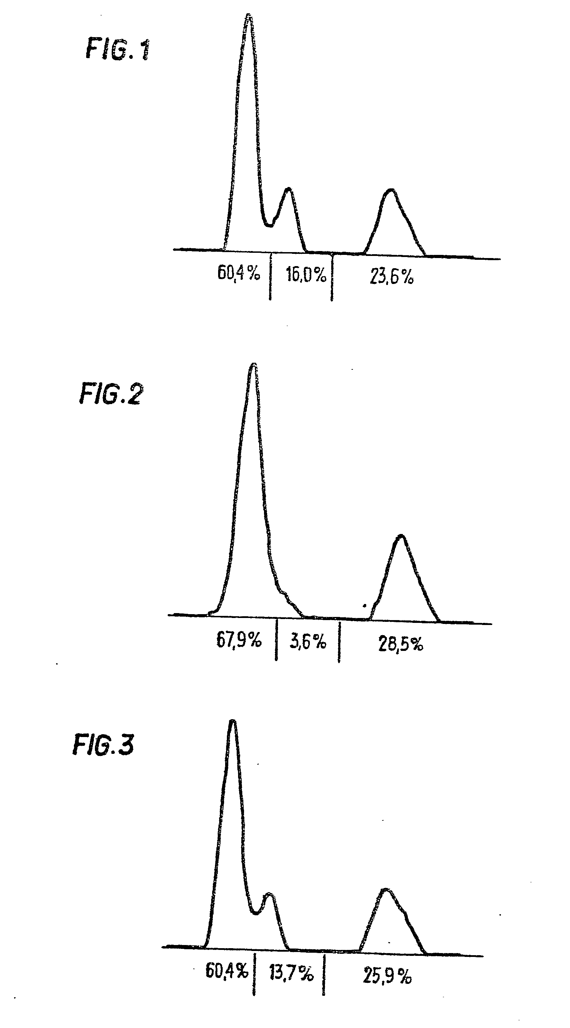

Fig. 1 zeigt ein Elektroferogramm eines frisch abgenommenen Nativserums, Fig. 2 ein Elektroferogramm desselben Serums, welches unmittelbar nach Abnahme gefroren und lyophilisiert und vor der Bestimmung eine Woche ge-

0022128

lagert wurde. Fig. 3 zeigt ein Elektroferogramm eines erfindungsgemäß unter Zugabe von 12 % w/v Saccharose konservierten und während eines Jahres gelagerten Serums.

In der Auswertung gemäß Fig. 1 sind die Gehalte an $\beta$-Lipoprotein, Prä-$\beta$-Lipoprotein und $\alpha$-Lipoprotein durch den Schreiber eines Densitometers in nativer Form unmittelbar nach Blutabnahme dargestellt. Der erste Peak gibt die Relativprozente von $\beta$-Lipoprotein mit 60,4 %, der zweite Peak die von Prä-$\beta$-Lipoprotein mit 16,0 % und der dritte Peak die von $\alpha$-Lipoprotein mit 23,6 % an.

Aus Fig. 2 ist ersichtlich, daß sich das Verhältnis von $\beta$-Lipoprotein zu Prä-$\beta$-Lipoprotein nach einwöchiger Lagerung verändert hat. Der Prä-$\beta$-Lipoprotein-Peak ist beinahe ganz verschwunden. Die Integratorauswertung ergibt verfälscht 67,9 % $\beta$-Lipoprotein, 3,6 % Prä-$\beta$-Lipoprotein und 28,5 % $\alpha$-Lipoprotein wieder.

Bei dem nach erfindungsgemäßer Behandlung erhaltenen Elektroferogramm gemäß Fig. 3 hingegen ist der Prä-$\beta$-Lipoprotein-Peak erhalten geblieben und die Integratorauswertung ergibt 60,4 % $\beta$-Lipoprotein, 13,7 % Prä-$\beta$-Lipoprotein und 25,9 % $\alpha$-Lipoprotein - sehr nahe der ursprünglichen Zusammensetzung.

Patentansprüche:

1. Verfahren zum Konservieren der elektrophoretischen Eigenschaften von Lipoproteinen, insbesondere von LDL-, VLDL- und HDL-Lipoproteinen in menschlichem oder tierischem Plasma, Plasmafraktionen oder Seren, in welchen das Lipoproteinmuster elektrophoretisch zu bestimmen ist, dadurch gekennzeichnet, daß dem Plasma bzw. den Plasmafraktionen oder Seren nicht-reduzierende Zucker, insbesondere Saccharose, zugegeben werden, das Gemisch lyophilisiert und das Lyophilisat vor der Bestimmung rekonstituiert wird.

2. Verfahren nach Anspruch 1, dadurch gekennzeichnet, daß die nicht-reduzierenden Zucker in einer Menge von 10 bis 20 % w/v zugegeben werden.

3. Verfahren nach Anspruch 1 oder 2, dadurch gekennzeichnet, daß das Zucker enthaltende Gemisch vor der Lyophilisierung bei mindestens -70°C schockgefroren wird, worauf nach Erwärmen auf -40°C bis -35°C das Gemisch lyophilisiert wird.

4. Anwendung von nach den Ansprüchen 1 bis 3 konservierten Seren als Lipoprotein-Elektrophorese-Kontrollseren für Plasma- bzw. Serumuntersuchungen.

0022128

1/1

FIG.1

60,4% | 16,0% | 23,6%

FIG.2

67,9% | 3,6% | 28,5%

FIG.3

60,4% | 13,7% | 25,9%

Europäisches
Patentamt

**EUROPÄISCHER RECHERCHENBERICHT**

Nummer der Anmeldung

EP 80 89 0068.2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A1 - 2 727 730 (TECHNICON INSTRUMENTS CORP.) <br> * ganze Druckschrift * <br> -- | |
| A | US - A - 4 045 176 (G.J. PROKSCH et al.) <br> * ganze Druckschrift * <br> -- | |
| A,D | US - A - 4 127 502 (CH.M. LI MUTTI et al.) <br> * ganze Druckschrift * <br> & DE - A1 - 2 825 391 <br> -- | |
| A,D | AT - B - 320 865 (IMMUNO AKTIENGESELL-SCHAFT FÜR CHEMISCH-MEDIZINISCHE PRODUKTE) <br> * ganze Druckschrift * <br> -- | |
| A,P | US - A - 4 188 188 (H. WILLNER et al.) <br> * ganze Druckschrift * <br> ---- | |

**KLASSIFIKATION DER ANMELDUNG (Int.Cl.3)**

G 01 N 33/92

G 01 N 33/68

G 01 N 33/96

**RECHERCHIERTE SACHGEBIETE (Int. Cl.3)**

G 01 N 33/50

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patent-familie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 22-09-1980 | SCHWARTZ |

EPA form 1503.1 06.78